# EUROPEAN PATENT SPECIFICATION

(11) **EP 2 060 237 B1**
(45) Date of publication and mention of the grant of the patent: **19.10.2011**
(21) Application number: 07425713.0
(22) Date of filing: 13.11.2007
(51) Int. Cl.: A61B 17/68, A61B 17/72

(54) **Removable closure unit for axial bone consolidation implants**
Entfernbare Verschlusseinheit für axiale Knochenkonsolidierungsimplantate
Unité de fermeture amovible pour implants de consolidation d'os axial

(43) Date of publication of application: 20.05.2009
(73) Proprietor: CITIEFFE S.r.l., 40012 Calderara di Reno (Bologna) (IT)
(72) Inventor: Mingozzi, Franco, 40012 Lippo di Calderara di Reno (Bologna) (IT); Dovesi, Alan, 40100 Bologna (IT)
(74) Representative: Modiano, Micaela Nadia

(56) References cited:
- WO-A-89/07056
- WO-A-92/03979
- DE-U1- 29 818 323

## Description

The present invention relates to a removable closure unit for axial bone consolidation implants: the unit is particularly suitable for closing nails of the ENDOVIS type used in the surgical technique that bears the same name, adapted to repair fractures of the femur (lateral fractures of the neck of the femur, long-fissure subtrochanteric fractures, fractures of the lateral region of the neck of the femur associated with diaphyseal fractures).

The fracture of the neck of the femur is certainly the most severe among fractures which occur due to osteoporosis.

Since the proximal portion of the femur is subjected to considerable stresses even in young individuals, it is understandable to think that during osteoporosis it is one of the structures that has the highest risk of fracture. The neck of the femur normally bears compression loads produced both by body weight and by muscular force. In the upright position, approximately one third of body weight is supported by the two hips. This increases if the weight is transferred to a single leg. In this case, the entire body weight receives the addition of increased muscular forces and the reaction force of the joints increases the body weight by 2.5-3 times.

The proximal portion of the femur is capable of bearing these loads in normal conditions. The elliptical shape of the neck of the femur increases its ability to bear upper and lower moments of flexure.

As age increases, the mineral density of the neck of the femur decreases more in women than in men. This reduction in bone mass is associated with a reduction in the strength of the bone, which is greater in women.

In case of fracture, caused by external traumas or induced by such traumas, of a femur which has already been weakened by osteoporosis, it is necessary to resort to surgical treatments which provide for osteosynthesis.

One interesting means of osteosynthesis is the "Endovis nail", the design of which dates back to 1993. Substantially, with respect to the traditional nail, it is provided with two parallel head screws. The proximal head screw is larger, while the underlying one is smaller. Both screws have such characteristics as to ensure a good portion of surface of contact with the spongy bone of the neck of the femur. Their implantation has the advantage of avoiding mainly the rotational instability of the femoral head.

The Endovis nail is hollow, and for this reason, once the procedure for its implantation has ended, it is necessary to block its proximal end by means of a removable plug.

Known types of plug comprise a threaded front end portion which is adapted to mate stably with a complementary thread provided inside the proximal end of the nail.

A seat is provided on the rear face of known plugs and is conveniently shaped for mating with a tool suitable for screwing the plug onto the proximal end of the nail.

The space available to surgeons to screw the plug is minimal, and insertion of the plug in alignment such as to allow easy and immediate screwing is definitely complicated.

In practice, a considerable amount of time and several attempts are necessary in order to achieve the correct arrangement of the plug which ensures stable and durable screwing.

This complication increases the time required for the surgical procedure, which in practice is not always performed perfectly, with the risk that the plug may subsequently exit from the nail.

WO 89/07056 A discloses an intramedullary nail comprising a combination of elements as set forth in the pre-characterizing portion of the appended claim 1.

The aim of the present invention is to provide a removable closure unit for axial bone consolidation implants which is simple and convenient to insert in the corresponding implant.

Within this aim, an object of the present invention is to provide a removable closure unit for axial bone consolidation implants which can be removed easily even by adopting tools of a standard type.

Another object of the present invention is to provide a removable closure unit for axial bone consolidation implants which is particularly stable in order to avoid unintentional exit from the seat of the implant within which it is installed.

Another object of the present invention is to provide a removable closure unit for axial bone consolidation implants which has a low cost, is relatively simple to provide in practice and safe in application.

In accordance with the invention, there is provided a removable closure unit for axial bone consolidation implants, as defined in the appended claims.

Further characteristics and advantages of the invention will become better apparent from the following detailed description of a preferred but not exclusive embodiment of a removable closure unit for axial bone consolidation implants, illustrated by way of nonlimiting example in the accompanying drawings, wherein:

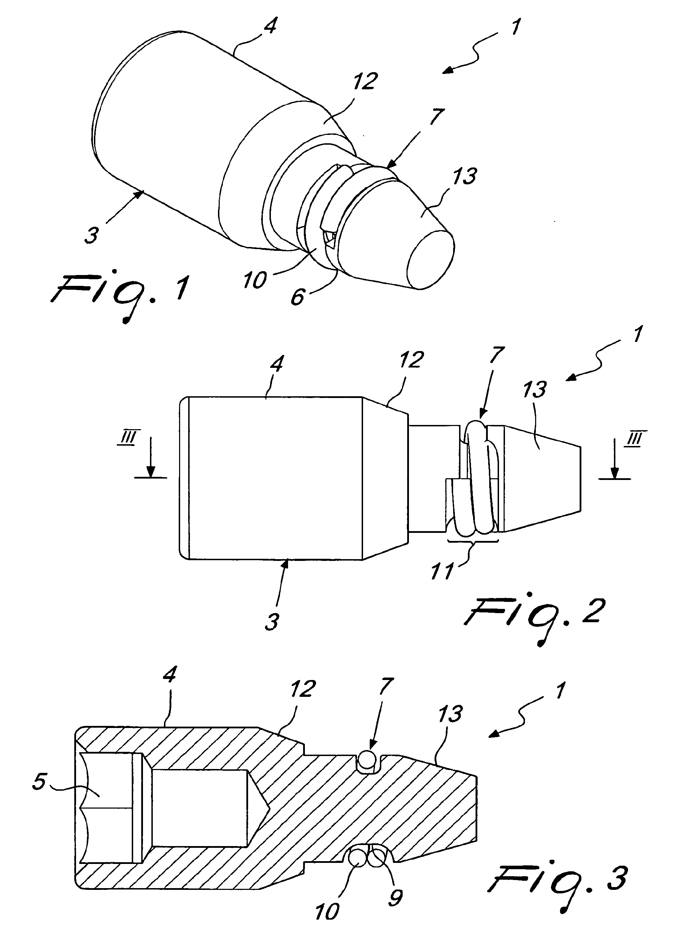

Figure 1 is a perspective view of a unit according to the invention;
Figure 2 is a side view of a unit according to the invention;
Figure 3 is a sectional view, taken along the line III-III outlined in Figure 2, of a unit according to the invention;

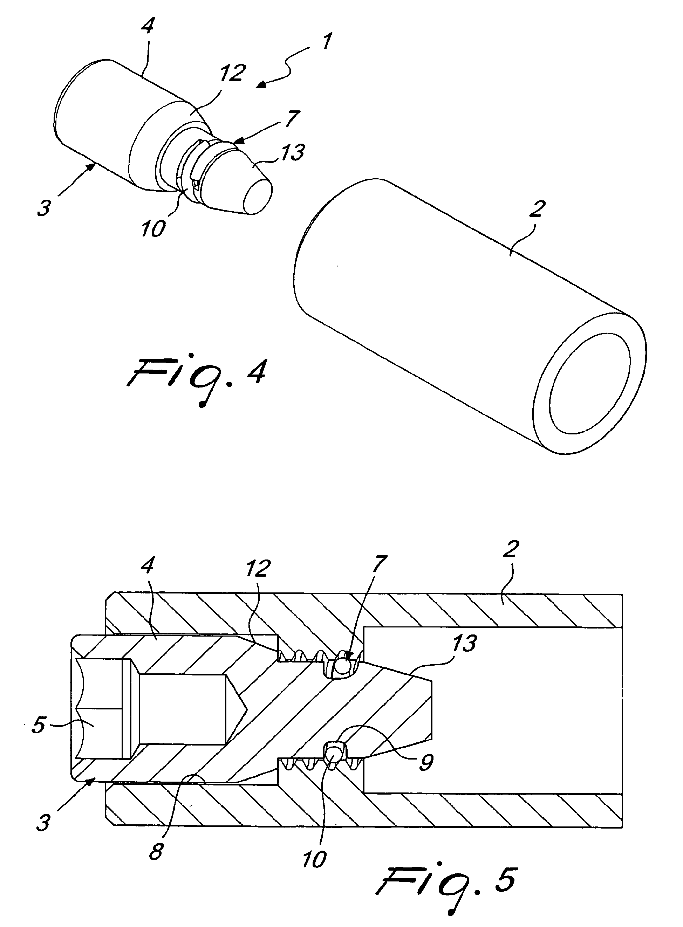

Figure 4 is a perspective view of a unit according to the invention, aligned with a respective implant;
Figure 5 is a sectional view, taken along an axial plane, of a unit according to the invention, coupled to a respective implant.

With reference to the figures, the reference numeral 1 generally designates a removable closure unit for axial implants 2 for bone consolidation.

The unit 1 comprises a main body 3 which is substantially axially symmetrical and is provided, at a first end 4, with a seat 5 for coupling to a movement tool and, at a second end 6, with coupling elements 7 with a suitable terminal receptacle 8 of the corresponding axial implant 2.

The coupling elements 7 comprise a groove 9 which has a substantially helical shape and a stem 10 which also is substantially helical and shaped complementarily with respect to the groove 9 and is accommodated with play within the groove 9.

The stem 10 can be deformed elastically from a free configuration in which it protrudes outward (a configuration which the stem 10 assumes normally and therefore in the absence of external actions) with respect to the groove 9, to a configuration in which it is fully inserted within the groove 9 as a consequence of deformations caused by radially oriented external actions (substantially, deformations caused by a compression of the stem 10 toward the bottom of the groove 9).

In order to achieve the two extreme configurations of the stem 10, the width and depth of the groove 9 must not be substantially smaller than the diameter of the stem 10: in this manner, the stem 10 can in fact be accommodated completely within the groove 9 in case of deforming actions imparted from outside.

According to an embodiment of particular interest in practice and in application, the groove 9 is shaped like a helix whose pitch is smaller than the width of the groove 9, forming a continuous recess 11 which corresponds to the envelope area of the generating helix.

The axially symmetrical body 3 comprises, in the first end 4, a bush which is provided with an internal seat 5 whose shape and dimensions are complementary to those of a tool designed to move the body 3, and an area 12 for blending with the second end 6.

The axially symmetrical body 3 comprises, in the second end 6, a tip 13, which substantially tapers in order to facilitate its insertion within the corresponding axial implant 2, and a substantially cylindrical central region.

The substantially cylindrical central region is the one on which the groove 9 is provided.

It is convenient to note that in order to be able to utilize the best possible mechanical properties, it is advisable to adopt stems 10 and bodies 3 made of metallic material, for example surgical steel.

To use the unit 1 according to the invention it is necessary first of all to insert the coupling end of a tool in the seat 5 of the first end 4 of the main body 3.

It is then necessary to align, by means of such tool, the second end 6 with the terminal receptacle 8 of the corresponding axial implant 2 inserted within a bone of a patient.

At this point, the operator has to push the second end 6 into the implant 2, in a respective threaded cavity, so as to force the stem 10 to retract into the groove 9 until a terminal abutment is reached.

In these conditions, the stem 10 can again protrude from the groove 9, since it is accommodated within a corresponding start of the thread of the cavity of the implant 2.

A rotation of the unit 1 when it is inserted within the receptacle 8, performed in the unscrewing direction (usually counterclockwise) with the tool, entails the abutment of the end face of the stem 10 against the front of the groove 9 in mutual locking conditions.

This entails a sliding of the stem 10 along the internal thread of the implant 2, with consequent exit by unscrewing of the unit 1 from the implant 2.

In practice, the unit 1 according to the invention allows easier insertion by pressing in the corresponding implant 2, although it can be extracted easily by unscrewing it as occurs with known types of embodiment.

With the unit 1 according to the invention, there is proposed a method for using the removable closure unit for the axial implants 2 for bone consolidation, that comprises the steps of: inserting the coupling end of a tool in the seat 5 of the first end 4 of the main body 3 of the unit 1; aligning, by means of the tool, the second end 6 of the main body 3 with the receptacle 8 of the corresponding axial consolidation implant 2 inserted within a bone of a patient; pushing the second end 6 into the implant 2, into a respective threaded cavity of the receptacle 8, so as to force the deformable stem 10 accommodated within the groove 9 to retract therein until a terminal abutment is reached at which the stem 10 can again exit from the groove 9, being accommodated within a corresponding start of the thread of the cavity of the implant 2. Moreover, such method may further include that a rotation imparted to the unit 1 when it is inserted within the receptacle 8 in the unscrewing direction, performed with the tool, entails the abutment of the end face of the stem 10 against the front of the groove 9 in mutual locking conditions and therefore a sliding of the stem 10 along the internal thread of the implant 2, with consequent exit by unscrewing of the unit 1 from the implant 2.

It has thus been shown that the invention achieves the intended aim and objects.

The invention thus conceived is susceptible of numerous modifications and variations, all of which are within the scope of the appended claims.

All the details may further be replaced with other technically equivalent elements.

In the exemplary embodiments shown, individual characteristics, given in relation to specific examples, may actually be interchanged with other different characteristics that exist in other exemplary embodiments.

In practice, the materials used, as well as the shapes and dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly such reference signs do not have any limiting effect on the interpretation of each element identified by way of example by such reference signs.

## Claims

1. A removable closure unit for axial bone consolidation implants (2), comprising a main body (3) which is substantially axially symmetrical and is provided, at a first end (4), with a seat (5) for coupling to a movement tool and, at a second end (6), with elements (7) for coupling to a terminal receptacle (8) of the corresponding axial implant (2), **characterized in that** said coupling elements (7) comprise a groove (9) which has a substantially helical shape and a stem (10) which also has a substantially helical shape which is complementary with respect to said groove (9) and is accommodated with play within said groove (9), said stem (10) being elastically deformable from a free configuration, in which it protrudes outward with respect to said groove (9), to a configuration deformed by radially oriented external actions, in which it is completely inserted within said groove (9).

2. The unit according to claim 1, **characterized in that** said groove (9) has a width and depth which are substantially not smaller than the diameter of said stem (10).

3. The unit according to claim 1, **characterized in that** said groove (9) is shaped like a helix which has a pitch which is smaller than the width of said groove (9), forming a continuous recess (11) which corresponds to the envelope area of the generating helix.

4. The unit according to claim 1, **characterized in that** said axially symmetrical body (3) comprises, in the first end (4), a bush provided with an internal seat (5) whose shape and dimensions are complementary to those of a tool designed to move said body (3), and an area (12) for blending with said second end (6).

5. The unit according to claim 1, **characterized in that** said axially symmetrical body (3) comprises, in the second end (6), a tip (13) which tapers substantially in order to facilitate its insertion within the corresponding axial implant (2) and a substantially cylindrical central region.

6. The unit according to the preceding claim, **characterized in that** said substantially cylindrical central region comprises said groove (9).

7. The unit according to claim 1, **characterized in that** said stem (10) is made of metallic material.

8. The unit according to claim 1, **characterized in that** said main body (3) is made of metallic material.

## Patentansprüche

1. Entfernbare Verschlusseinheit für axiale Knochenkonsolidierungsimplantate (2), mit einem Hauptkörper (3), welcher im Wesentlichen axialsymmetrisch ist und an einem ersten Ende (4) mit einem Sitz (5) zum Koppeln an ein Bewegungswerkzeug und an einem zweiten Ende (6) mit Elementen (7) zum Koppeln an eine Endaufnahme (8) des zugeordneten axialen Implantats (2) versehen ist, **dadurch gekennzeichnet, dass** die Kopplungselemente (7) eine Nut (9), welche eine im Wesentlichen schraubenförmige Form hat, und einen Steg (10) aufweisen, welcher ebenfalls eine im Wesentlichen schraubenförmige Form hat, welche in Bezug auf die Nut (9) komplementär ist, und mit Spiel innerhalb der Nut (9) aufgenommen ist, wobei der Steg (10) aus einer freien Konfiguration, in welcher er in Bezug auf die Nut (9) nach außen vorsteht, elastisch in eine Konfiguration verformbar ist, die durch radial gerichtete externe Einwirkungen verformt wird, bei welchen er vollständig in die Nut (9) eingelegt wird.

2. Einheit nach Anspruch 1, **dadurch gekennzeichnet, dass** die Nut (9) eine Breite und eine Tiefe hat, welche im Wesentlichen nicht kleiner als der Durchmesser des Stegs (10) sind.

3. Einheit nach Anspruch 1, **dadurch gekennzeichnet, dass** die Nut (9) wie eine Helix geformt ist, welche eine Steigung hat, welche kleiner als die Breite der Nut (9) ist, die eine durchgehende Ausnehmung (11) bildet, welche der Hüllkurvenfläche der erzeugenden Helix entspricht.

4. Einheit nach Anspruch 1, **dadurch gekennzeichnet, dass** der axialsymmetrische Körper (3) in dem ersten Ende (4) eine mit einem inneren Sitz (5) versehene Buchse, dessen Form und Abmessungen komplementär zu denen eines Werkzeugs sind, das zum Bewegen des Körpers (3) vorgesehen ist, und eine Fläche (12) für den Übergang auf das zweite Ende (6) aufweist.

5. Einheit nach Anspruch 1, **dadurch gekennzeichnet, dass** der axialsymmetrische Körper (3) in dem zweiten Ende (6) eine Spitze (13), die im Wesentlichen spitz zuläuft, um deren Einführen in das entsprechende axiale Implantat (2) zu erleichtern, und einen im Wesentlichen zylindrischen mittleren Bereich aufweist.

6. Einheit nach dem vorhergehenden Anspruch, **dadurch gekennzeichnet, dass** der im Wesentlichen zylindrische mittlere Bereich die Nut (9) aufweist.

7. Einheit nach Anspruch 1, **dadurch gekennzeichnet, dass** der Steg (10) aus metallischem Material hergestellt ist.

8. Einheit nach Anspruch 1, **dadurch gekennzeichnet, dass** der Hauptkörper (3) aus metallischem Material hergestellt ist.

## Revendications

1. Unité de fermeture amovible pour implants de consolidation osseuse axiale (2), comprenant un corps principal (3) qui est sensiblement axialement symétrique et est muni, à une première extrémité (4), d'un siège (5) pour coupler à un outil de mouvement et, à une seconde extrémité (6), d'éléments (7) pour coupler à un réceptacle terminal (8) de l'implant axial correspondant (2), **caractérisé en ce que** lesdits éléments de couplage (7) comprennent une rainure (9), qui a une forme sensiblement hélicoïdale, et une tige (10), qui a également une forme sensiblement hélicoïdale, complémentaire de ladite rainure (9) et reçue avec un certain jeu dans ladite rainure (9), ladite tige (10) étant élastiquement déformable d'une configuration libre, dans laquelle elle fait saillie vers l'extérieur par rapport à ladite rainure (9), à une configuration déformée par des actions externes radialement orientées, où elle est complètement insérée dans ladite rainure (9).

2. Unité selon la revendication 1, **caractérisée en ce que** ladite rainure (9) a une largeur et une profondeur qui ne sont sensiblement pas inférieures au diamètre de ladite tige (10).

3. Unité selon la revendication 1, **caractérisée en ce que** ladite rainure (9) est conformée en hélice qui a un pas inférieur à la largeur de ladite rainure (9), formant un évidement continu (11) qui correspond à la zone d'enveloppe de l'hélice génératrice.

4. Unité selon la revendication 1, **caractérisée en ce que** ledit corps axialement symétrique (3) comprend, à la première extrémité (4), un manchon muni d'un siège interne (d), dont la forme et les dimensions sont complémentaires de celles d'un outil conçu pour déplacer ledit corps (3), et une zone (12) pour se fondre ladite deuxième extrémité (6).

5. Unité selon la revendication 1, **caractérisée en ce que** ledit corps axialement symétrique (3) comprend, à la seconde extrémité (6), une pointe (13), qui s'amincit sensiblement afin de faciliter son insertion dans l'implant axial correspondant (2), et une région centrale sensiblement cylindrique.

6. Unité selon la revendication précédente, **caractérisée en ce que** ladite région centrale sensiblement cylindrique comprend ladite rainure (9).

7. Unité selon la revendication 1, **caractérisée en ce que** ladite tige (10) est formée d'un matériau métallique.

8. Unité selon la revendication 1, **caractérisée en ce que** ledit corps principal (3) est formé d'un matériau métallique.
